(19) **Europäisches Patentamt**

**European Patent Office**

**Office européen des brevets**

(11) **EP 0 678 301 B2**

(12) **NOUVEAU FASCICULE DE BREVET EUROPEEN**

| | |
|---|---|
| (45) Date de publication et mention de la décision concernant l'opposition: **03.05.2006 Bulletin 2006/18** | (51) Int Cl.: *A61M 1/16* (2006.01) *A61M 1/34* (2006.01) |

(45) Mention de la délivrance du brevet: **04.08.1999 Bulletin 1999/31**

(21) Numéro de dépôt: **95202060.0**

(22) Date de dépôt: **09.09.1992**

(54) **Appareil multifonction pour le traitement de l'insuffisance rénale**

Multifunktionelles Gerät zur Behandlung von Niereninsuffizienz

Multifunctional apparatus for treatment of renal insufficiency

(84) Etats contractants désignés:
**AT BE CH DE ES FR GB IT LI NL SE**

(30) Priorité: **10.09.1991 FR 9111351**

(43) Date de publication de la demande:
**25.10.1995 Bulletin 1995/43**

(62) Numéro(s) de document de la (des) demande(s) initiale(s) en application de l'article 76 CBE:
**92420299.7 / 0 532 432**

(73) Titulaire: **Gambro Industries SAS**
**69330 Meyzieu (FR)**

(72) Inventeurs:
• **Chevallet, Jacques**
**F-69360 Serezin du Rhône (FR)**
• **Bene, Bernard**
**F-69540 Irigny (FR)**

• **Lobdell Donn D.**
**Corona Del Mar, CA 92625 (US)**
• **Corbin III Frank**
**Littleton, CO 80123 (US)**
• **Leppert Lawrence L.**
**Littleton, CO 80123 (US)**
• **Johnson Steven H.**
**Lakewood, CO 80226 (US)**

(74) Mandataire: **Altenburg, Udo**
**Patent- und Rechtsanwälte**
**Bardehle . Pagenberg . Dost .**
**Altenburg - Geissler**
**Galileiplatz 1**
**81679 München (DE)**

(56) Documents cités:
**EP-A- 0 321 754** **EP-A- 0 373 455**
**DE-A- 2 552 304** **FR-A- 2 397 197**
**US-A- 4 670 007** **US-A- 4 769 132**
**US-A- 4 889 528**

**EP 0 678 301 B2**

## Description

La présente invention a pour objet un appareil permettant de soumettre à différents types de traitement des patients ayant perdu l'usage de leurs reins, en particulier à la suite d'un accident ou d'une opération chirurgicale.

Le document FR 2 397 197 décrit un appareil au moyen duquel il est possible de soumettre un patient simultanément à un traitement par dialyse et à un traitement par hémofiltration. Cet appareil comprend notamment :

- un échangeur ayant deux compartiments séparés par une membrane semi-perméable, un premier compartiment étant relié à un circuit pour circulation extracorporelle de sang connectable à un patient, un second compartiment ayant une entrée et une sortie ;
- des premiers moyens de pompage pour faire circuler un premier liquide dans une canalisation reliée à une entrée du second compartiment ;
- des seconds moyens de pompage pour faire circuler un second liquide dans une canalisation reliée au circuit pour circulation extracorporelle de sang ;
- des troisièmes moyens de pompage pour faire circuler du sang dans le circuit pour circulation extracorporelle de sang ; et
- des moyens d'extraction pour provoquer l'ultrafiltration d'un liquide corporel au travers de la membrane de l'échangeur.

Accessoirement, l'invention a pour objet un appareil permettant le dosage précis, dans le sang, de substances telles que des médicaments (antibiotiques, notamment), du glucose, ou certains électrolytes du sang (potassium, magnésium, bicarbonate, en particulier). Dans la suite, on décrira l'invention dans son application au dosage du bicarbonate, mais il va de soi que cet exemple d'utilisation, donné à titre purement illustratif, ne saurait être considéré comme limitatif.

On sait que les reins, outre des fonctions d'épuration des déchets plasmatiques (urée, créatinine) et d'excrétion hydrique, jouent un rôle important dans le maintien de l'équilibre acido-basique du milieu interne, notamment en éliminant des acides faibles (phosphates, acides monosodiques) et en produisant des sels d'ammonium.

Chez les personnes qui ont perdu l'usage de leurs reins, temporairement ou définitivement, ce mécanisme régulateur ne jouant plus, on constate une élévation de l'acidité du milieu intérieur (acidose), c'est-à-dire aussi une baisse du pH du sérum sanguin, qui se déplace vers 7 (le pH sanguin varie normalement dans les limites très étroites qui vont de 7,35 à 7,45).

De façon classique, pour pallier la déficience du mécanisme régulateur rénal, on agit sur un autre mécanisme régulateur de l'équilibre acido-basique du milieu interne, qui est constitué par les systèmes tampons du sang, dont le principal comprend l'acide carbonique, comme acide faible, associé à son sel alcalin, le bicarbonate. C'est ainsi que, pour combattre l'acidose d'une personne souffrant d'insuffisance rénale, on fait passer du bicarbonate dans son sang, généralement simultanément à une séance d'épuration du sang par hémodiafiltration ou par hémodialyse.

Lors d'un traitement par hémofiltration, où le sang est épuré par ultrafiltration d'eau plasmatique au travers d'une membrane semi-perméable avec transfert convectif simultané de déchets plasmatiques, l'apport en bicarbonate se fait par perfusion d'une solution de bicarbonate de sodium.

Lors d'un traitement par hémodialyse, où le sang est épuré par transfert diffusif de déchets plasmatiques au travers d'une membrane semi-perméable dont la face non baignée par le sang est irriguée par un flux de liquide de dialyse, l'apport en bicarbonate peut se faire de deux façons, selon que le liquide de dialyse contient du bicarbonate ou qu'il en est dépourvu.

Dans le premier cas, l'apport au sang de bicarbonate se fait par diffusion au travers de la membrane semi-perméable, du liquide de dialyse vers le sang, la concentration en bicarbonate dans le liquide de dialyse étant ajustée en conséquence.

Dans le second cas, comme lors du traitement par hémofiltration, une solution de bicarbonate de sodium est perfusée au patient en quantité suffisante pour compenser les pertes diffusives (ou convectives, en hémofiltration) qui se produisent dans l'échangeur à membrane ainsi que le déficit dont souffre le malade en état d'acidose.

La concentration finale en bicarbonate du sang d'un patient soumis à l'un de ces traitements dépend de la concentration en bicarbonate de la solution de perfusion ou du liquide de dialyse, de leurs débits respectifs, et du débit du sang du patient circulant dans un circuit pour circulation extracorporelle de sang comprenant l'échangeur à membrane. Actuellement, à l'exception de la concentration de la solution de bicarbonate de sodium, qui est fixée par le fabricant, ces paramètres sont déterminés empiriquement par le médecin, à partir de mesures du pH sanguin faites régulièrement dans le cas des patients en état de choc, dont le sang est dialysé ou ultrafiltré en permanence, ou faites après une séance de traitement et avant la séance suivante, dans le cas des patients ayant définitivement perdu l'usage de leurs reins. Il en résulte que la concentration en bicarbonate du sang du patient correspond rarement exactement à la concentration souhaitée.

Un but de l'invention est de réaliser un appareil de traitement du sang qui soit particulièrement adapté à la mise en oeuvre continue de traitements multiples, et qui permette une commande et un contrôle précis des débits des liquides médicaux et corporels mis en circulation.

Selon l'invention, pour atteindre ce but, on prévoit un appareil multifonction pour la circulation de liquides corporels et médicaux selon la revendication 1.

Les premiers moyens de pesage comprennent soit deux moyens de pesage indépendants pour peser respectivement le premier réservoir et le second réservoir soit des moyens de pesage uniques pour peser ensemble le premier réservoir et le second réservoir.

Selon une caractéristique de l'invention, l'appareil comprend en outre des moyens de connexion pour rendre le premier réservoir sélectivement connectable à l'entrée du second compartiment de l'échangeur ou au circuit pour circulation extracorporelle de sang.

Selon une autre caractéristique de l'invention, l'appareil comprend en outre des moyens de dosage pour ajuster à une concentration souhaitée $[A]_{DES}$ dans le sang du patient la concentration d'une substance (A) présente dans le second liquide stérile, compte tenu du transfert diffusif et/ou convectif de la substance (A) au travers de la membrane de l'échangeur.

Selon une variante de l'invention, les moyens de dosage comprennent des moyens pour régler le débit $(Q_A)$ du second liquide stérile en fonction du débit $(Q_{OUT})$ de liquide usé, le débit $(Q_A)$ du second liquide stérile et le débit $(Q_{OUT})$ du liquide usé étant liés par la formule :

$$Q_A = \frac{[A]_{DES}}{[A]_{SOL}} \times Q_{OUT}$$

où $[A]_{SOL}$ est la concentration de la substance (A) dans le premier liquide stérile.

Selon une autre variante de l'invention, les moyens de dosage comprennent des moyens pour régler le débit $(Q_A)$ du second liquide stérile en fonction de la clairance Cl du rein artificiel pour la substance (A), le débit $(Q_A)$ du second liquide stérile et la clairance Cl étant liés par la formule :

$$Q_A = \frac{[A]_{DES}}{[A]_{SOL}} \times Cl$$

où $[A]_{SOL}$ est la concentration de la substance (A) dans le premier liquide stérile.

D'autres caractéristiques et avantages de l'invention apparaîtront à la lecture de la description qui va suivre. On se reportera aux dessins annexés sur lesquels :

la figure 1 est un schéma simplifié d'un premier mode de réalisation de l'invention.
la figure 2 est un schéma simplifié d'un second mode de réalisation de l'invention.

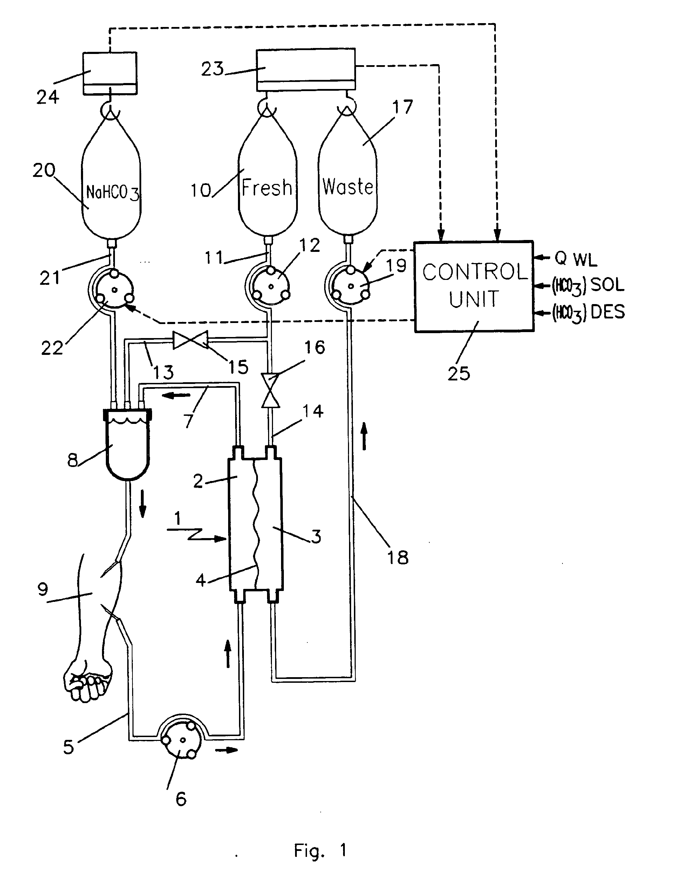

Le rein artificiel représenté sur la figure 1 comprend un échangeur ayant deux compartiments 2, 3 séparés par une membrane semi-perméable 4. Le compartiment 2 est connecté à un circuit pour circulation extracorporelle de sang comprenant une canalisation amont 5, sur laquelle est disposée une pompe de circulation 6, et une canalisation aval 7 équipée d'un piège à bulles 8. Les canalisations 5 et 7 sont respectivement munies à leur extrémité libre d'une aiguille ou d'un connecteur pour cathéter, pour le raccordement du circuit pour circulation extracorporelle de sang au système vasculaire d'un patient 9.

Un réservoir 10 de liquide de dialyse/substitution stérile et dépourvu de bicarbonate est relié, par l'intermédiaire d'une portion de canalisation commune 11 sur laquelle est disposée une pompe de circulation 12, à deux canalisations 13, 14 connectées respectivement au piège à bulles 8 et à une entrée du second compartiment 3 de l'échangeur 1. Des moyens d'occlusion 15, 16, tels que des camps électromagnétiques, sont prévus respectivement sur les canalisations 13, 14 pour permettre de relier sélectivement le réservoir 10 à l'échangeur 1 ou au circuit pour circulation extracorporelle de sang.

Un second réservoir 17 pour liquide usé (ultrafiltrat et/ou liquide de dialyse usé) est relié à une sortie du second compartiment 3 de l'échangeur 1, par une canalisation 18 sur laquelle est disposée une pompe d'extraction 19 de liquide usé. La pompe 19 permet de créer une dépression variable dans le compartiment 3 de l'échangeur 1, c'est-à-dire aussi de faire varier la pression transmembranaire et, par conséquent, le débit d'ultrafiltration.

Un troisième réservoir 20 de solution stérile de bicarbonate de sodium est relié au piège à bulles 8, par l'intermédiaire d'une canalisation 21 sur laquelle est disposée une pompe de circulation 22.

Conformément à l'invention, le rein artificiel représenté sur la figure 1 comprend des moyens pour établir un bilan du (des) liquide(s) perfusé(s) au patient 9 et des liquides usés, pour programmer éventuellement une perte de poids souhaitée par extraction d'eau plasmatique en excès par rapport au(x) liquide(s) perfusé(s), et pour ajuster la concentration en bicarbonate du plasma du patient à une valeur déterminée. Ces moyens comprennent une première balance 23 pour peser le réservoir 10 de liquide de dialyse/substitution et le réservoir 17 de liquide usé, une seconde balance 24 pour peser le réservoir 20 de solution de bicarbonate de sodium, et une unité de commande 25 pouvant recevoir, comme signaux d'entrée, les informations fournies par les balances 23, 24, une consigne $Q_{WL}$ de débit de perte de poids souhaité, la valeur $[HCO_3]_{SOL}$ de la concentration en bicarbonate de la solution contenue dans le réservoir 20 et une consigne $[HCO_3]_{DES}$ de concentration du sang en bicarbonate souhaitée. L'unité de commande 25 est prévue pour piloter la pompe 19 d'extraction de liquide usé, compte tenu du débit de perte de poids désiré $Q_{WL}$ et du débit $Q_{IN}$ imposé à la pompe 12 de circulation du liquide de dialyse/substitution, et pour piloter la pompe 22 de perfusion de la solution de bicarbonate compte tenu du débit $Q_{OUT}$ de la pompe 19 d'extraction de liquide usé.

Conformément à l'invention, l'asservissement du débit $Q_{HCO3}$ de la pompe de perfusion 22 au débit $Q_{OUT}$ de la pompe d'extraction 19 peut être défini, quel que soit le type de traitement auquel est soumis le patient, hémofiltration avec ou sans perfusion de liquide de substitution, hémodialyse ou hémodiafiltration, par la formule :

$$Q_{HCO3} = Q_{OUT} \times \frac{[HCO_3]_{DES}}{[HCO_3]_{SOL}} \qquad (1)$$

Le fonctionnement du rein artificiel qui vient d'être décrit est le suivant :

En mode hémofiltration sans perfusion de liquide de substitution, les camps 15, 16 sont fermés, la pompe 12 de circulation du liquide de dialyse/substitution est à l'arrêt et les pompes 19, 22 d'extraction de filtrat sanguin et de perfusion de solution de bicarbonate tournent. L'unité de commande 25 règle en continu le débit $Q_{OUT}$ de la pompe d'extraction 19, mesuré au moyen de la balance 23, de façon que ce débit soit égal en permanence à la somme du débit $Q_{WL}$ de perte de poids souhaité et du débit $Q_{HCO3}$ de perfusion de la solution de bicarbonate, mesuré au moyen de la balance 24. L'unité de commande 25 règle aussi en continu le débit $Q_{HCO3}$ de la pompe 22 de solution de bicarbonate, en fonction de la concentration en bicarbonate $[HCO_3]_{DES}$ souhaitée du sang du patient, de la concentration $[HCO_3]_{SOL}$ de la solution contenue dans le réservoir 20, et de la perte convective se produisant dans l'échangeur 1, qui est égale à $Q_{OUT} \times [HCO_3]_{BLD}$, $[HCO_3]_{BLD}$ étant la concentration en bicarbonate du sang du patient et la transmittance des membranes haute perméabilité utilisées en hémafiltration étant égale à 1 pour les électrolytes du sang (on rappelle que la formule générale donnant le débit massique Js d'une substance traversant une membrane en fonction du débit volumétrique Jv d'eau plasmatique est la suivante

$$Js = Jv \times Tr \times Cs$$

où Cs est la concentration de la substance dans le sang et où Tr est la transmittance de la membrane vis à vis de cette substance.)

L'asservissement de la pompe 22 de perfusion de bicarbonate selon la formule (1) donnée plus haut permet donc d'amener progressivement le sang du patient 9 à un état d'équilibre où la concentration en bicarbonate est égale à $[HCO_3]_{DES}$.

En mode hémofiltration avec perfusion de liquide de substitution, le clamp 16 est fermé, le clamp 15 est ouvert et les trois pompes 12, 19, 22 tournent, le débit de la pompe 12 étant fixé par l'opérateur à une valeur constante en début de séance de traitement. Le fonctionnement du rein artificiel pour ce second mode de traitement ne diffère pas de celui qui vient d'être décrit plus haut, à ceci près que, pour piloter la pompe d'extraction 19, l'unité de commande 25 prend en compte la vidange du réservoir 10, le débit $Q_{OUT}$ imposé à la pompe 19 étant alors choisi pour que la différence entre le débit de liquide de substitution et le débit de liquide usé, mesurée par la balance 23, soit égale à la somme du débit $Q_{WL}$ de perte de poids souhaité et du débit $Q_{HCO3}$ de perfusion de solution de bicarbonate, mesuré par la balance 24. Le réglage de la pompe 22 de perfusion de solution de bicarbonate est effectué comme précédemment selon l'asservissement décrit par la formule (1).

En mode hémodialyse, le clamp 15 est fermé, le camp 16 est ouvert et les trois pompes 12, 19, 22 tournent. L'unité de commande 25 règle en continu le débit $Q_{OUT}$ de la pompe d'extraction 19, de façon que la différence entre le débit de liquide de dialyse et le débit de liquide usé, mesurée par la balance 23, soit égale en permanence au débit $Q_{HCO3}$ de perfusion de solution de bicarbonate, mesuré par la balance 24, la consigne de débit de perte de poids étant nulle.

L'unité de commande 25 règle en outre le débit $Q_{HCO3}$ de perfusion de la solution de bicarbonate, en fonction de la concentration en bicarbonate $[HCO_3]_{DES}$ souhaitée du sang du patient, de la concentration $[HCO_3]_{SOL}$ de la solution contenue dans le réservoir 20, et de la perte diffusive dans l'échangeur 1, qui est égale à $Cl \times [HCO_3]_{BLD}$, $[HCO_3]_{BLD}$ étant la concentration en bicarbonate du sang du patient et Cl, la clairance du rein artificiel pour le bicarbonate (la clairance est définie, de façon générale, comme le rapport entre la quantité de substance éliminée par unité de temps et la concentration de la substance dans le sang à l'entrée de l'échangeur). Pour obtenir que la concentration en bicarbonate du sang atteigne, à l'équilibre, une valeur donnée $[HCO_3]_{DES}$, il faut donc asservir le débit $Q_{HCO3}$ de perfusion de la pompe 22 de solution de bicarbonate selon la formule :

$$Q_{HCO3} = Cl \times \frac{[HCO_3]_{DES}}{[HCO_3]_{SOL}} \qquad (2)$$

ce qui suppose une détermination préalable de la clairance du rein artificiel, laquelle dépend du type d'échangeur utilisé (nature de la membrane, surface) ainsi que, en général, des débits du sang et du liquide de dialyse dans l'échangeur.

Cependant, pour certaines valeurs de débits de sang et de liquide de dialyse, la clairance du rein pour une substance et un type d'échangeur donnés est sensiblement constante. C'est le cas en particulier lorsque, d'une part, la surface de la membrane de l'échangeur est suffisamment grande par rapport au débit du sang, et que, d'autre part, le débit du sang est relativement important par rapport au débit du liquide de dialyse (rapport de l'ordre de trois, et plus). Le sang et le liquide de dialyse ont alors, au sortir de l'échangeur, la même concentration en la substance considérée, et la clairance Cl est égale au débit de sortie du liquide usé, $Q_{OUT}$. En d'autres termes, dans ces conditions de fonctionnement particulières, l'asservissement de la pompe 22 de perfusion de solution de bicarbonate est défini par la formule (1). Or, ces conditions sont applicables au traitement par dialyse continue des patients en état de choc dont l'épuration doit se faire à un taux modéré pour pouvoir être supportée par des organismes affaiblis.

Le rein artificiel selon l'invention présente donc un intérêt particulier pour le traitement des patients ayant perdu l'usage temporaire de leurs reins, puisque, quel que soit le type de traitement auxquels ils sont soumis, ce rein artificiel permet, par la commande d'une seule pompe selon une formule d'asservissement unique, d'agir simplement sur leur équilibre acido-basique.

Ce rein artificiel peut aussi fonctionner en mode hémodiafiltration, dans lequel la position des camps et le fonctionnement des pompes sont les mêmes que ceux qui sont adoptés en mode hémodialyse, à ceci près que la pompe 19 est pilotée pour provoquer une ultrafiltration dans le rein conformément à une consigne de débit de perte de poids donnée.

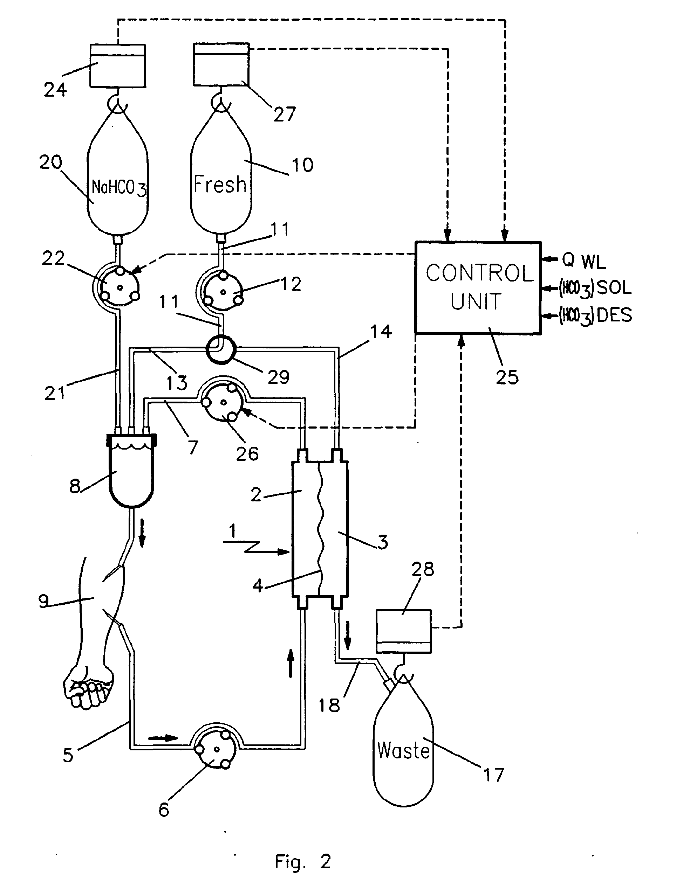

Le rein artificiel représenté sur la figure 2 se différencie de celui qui vient d'être décrit en ce que son circuit pour circulation extracorporelle de sang comprend une seconde pompe 26 disposée en aval de l'échangeur 1, pour permettre de faire varier la pression transmembranaire dans l'échangeur 1 et, par suite, le débit d'eau plasmatique ultrafiltrée (c'est-à-dire $Q_{OUT}$ en hémofiltration). Par ailleurs, les réservoirs 10, 17 de liquide de dialyse/substitution et de liquide usé sont pesés par des balances indépendantes 27, 28 et la canalisation 18 reliant le compartiment 3 de l'échangeur 1 au réservoir de liquide usé 17 est dépourvue de pompe.

En outre, une vanne trois voies 29, à laquelle sont raccordées les canalisations 11, 13 et 14, permet soit de relier le réservoir 10 de liquide de dialyse/substitution au piège à bulles 8, soit de relier le réservoir 10 au compartiment 3 de l'échangeur 1, soit d'isoler le réservoir 10.

Le fonctionnement de ce second mode de réalisation de rein artificiel selon l'invention ne diffère pas sensiblement de celui du précédent. En mode hémofiltration sans perfusion de liquide de substitution, la pompe 12 ne tourne pas et le débit de la pompe 26 est réglé par l'unité de commande 25 pour que le débit de filtration $Q_{OUT}$, mesuré par la balance 28, soit égal à la somme du débit de consigne de perte de poids $Q_{WL}$ et du débit de perfusion de la solution de bicarbonate $Q_{HCO3}$ mesuré par la balance 24.

En mode hémofiltration avec perfusion de liquide de substitution, la pompe 12 tourne à un débit réglé initialement par l'opérateur et le débit de la pompe 26 est asservi par l'unité de commande 25 pour que le débit de filtration $Q_{OUT}$ soit égal à la somme du débit de consigne de perte de poids $Q_{WL}$, du débit de perfusion de la solution de bicarbonate $Q_{HCO3}$ et du débit $Q_{IN}$ de perfusion du liquide de substitution, mesuré par la balance 27.

En mode de dialyse, les pompes 6 et 26 disposées sur le circuit sang, respectivement en amont et en aval de l'échangeur 1, tournent au même débit et la pompe 12, qui sert alors de pompe de circulation de liquide de dialyse, tourne à un débit réglé initialement par l'opérateur.

En mode hémodialfiltration, l'unité de commande 25 règle le débit de la pompe 26 comme en mode hémofiltration avec perfusion de liquide de substitution.

Sauf en mode hémofiltration, où elle ne tourne pas, le débit de la pompe 12 de circulation de liquide de dialyse/substitution est régulé par l'unité de commande 25 qui compare le débit souhaité mis initialement en mémoire dans cette unité et le débit mesuré au moyen de la balance 27. Quant à la pompe 22 de perfusion de bicarbonate, son débit $Q_{HCO3}$ est, comme précédemment, asservi au débit de liquide usé $Q_{OUT}$, mesuré par la balance 28, conformément à la formule (1) et, le cas échéant, à la formule (2).

L'invention n'est pas limitée aux modes de réalisation qui viennent d'être décrits et elle est susceptible de variantes.

En particulier, à la différence de ce qui est prévu dans les exemples de rein artificiel décrits plus haut, il est possible de prévoir que, dans les modes où du liquide de dialyse/substitution est mis en circulation par la pompe 12, ce soit le débit de la pompe 19 (26) commandant le débit d'ultrafiltration qui soit fixé initialement par l'opérateur et le débit de la pompe 12 qui soit asservi en fonction du bilan des liquides frais et usés, perfusés et ultrafiltrés et du débit de perte de poids souhaité.

Par ailleurs, la détermination du débit des liquides nécessaire à l'asservissement de la pompe 19 (26) réglant le débit d'ultrafiltration et à l'asservissement de la pompe 22 de perfusion de solution de bicarbonate, pourrait se faire avec d'autres moyens de mesure que des balances, par exemple avec des débitmètres ou des moyens de mesure volumétrique.

En outre, les pompes 12, 22 utilisées pour régler le débit des liquides de dialyse/substitution et de solution de bicarbonate pourraient être remplacées par des clamps électromagnétiques, les liquides s'écoulant par gravité.

Il va de soi aussi que la source 20 de liquide de perfusion pourrait être connectée directement au système vasculaire du patient 9 et non pas au circuit 5, 7 pour circulation extracorporelle de sang.

Enfin, comme cela a été mentionné plus haut, les moyens de dosage dont est équipé le rein artificiel selon l'invention peuvent être utilisés pour doser toutes sortes de substances dans le sang d'un patient soumis à une séance de traitement par hémofiltration, hémodialyse ou hémodialfiltration. Dans le cas d'un médicament A, par exemple, le réservoir 20 contient alors un soluté stérile de ce médicament, tandis que le réservoir 10 contient un liquide de dialyse dans lequel sont présents les principaux électrolytes du sang, bicarbonate compris. Le fonctionnement du rein n'est pas différent de celui qui a été décrit à propos des exemples de réalisation des figures 1 et 2, et, en particulier, l'asservissement de la pompe de perfusion 22 au débit de liquide usé se fait selon la formule (1), ou, le cas échéant, selon la formule (2).

**Revendications**

1. Appareil multifonction pour la circulation de liquides corporels et médicaux dans un échangeur (1) ayant deux compartiments (2, 3) séparés par une membrane semi-perméable (4), un premier compartiment (2) étant relié à un circuit (5, 7, 8) pour circulation extracorporelle de sang connectable à un patient (9), un second compartiment (3) ayant une entrée et une sortie, cet appareil comprenant :

   - des premiers moyens de pompage (12) pour faire circuler un premier liquide stérile ;
   - des seconds moyens de pompage (22) pour faire circuler un second liquide stérile ;
   - des troisièmes moyens de pompage (6, 26) pour faire circuler du sang dans le circuit pour circulation extracorporelle de sang (5, 7, 8) ;
   - des moyens d'extraction (19, 26) pour provoquer l'ultrafiltration d'un liquide corporel au travers de la membrane (4) de l'échangeur (1) ; cet appareil étant **caractérisé en ce qu'**il comprend :

      - des premiers moyens de pesage (23 ; 27, 28) pour peser un premier réservoir (10) pour le premier liquide stérile et un second réservoir (17) pour un liquide usé, le premier réservoir (10) étant connectable à l'entrée du second compartiment (3) de l'échangeur et le second réservoir (17) étant connectable à la sortie du second compartiment (3) de l'échangeur (1) ;
      - des seconds moyens de pesage (24) pour peser un troisième réservoir (20) pour le second liquide stérile, ce réservoir étant connectable au circuit extracorporel de sang (5, 7, 8) ; et
      - des moyens de commande (25) pour recevoir une information de poids des premiers moyens de pesage (23; 27, 28) et une information de poids des seconds moyens de pesage (24), et pour commander un des moyens parmi les premiers, les seconds, les troisièmes moyens de pompage (22, 12, 6. 26) et les moyens d'extraction (19, 26), à partir de ces informations de poids et d'au moins une information correspondant à un débit désiré d'au moins un liquide parmi le premier liquide stérile, le second liquide stérile, le sang et le liquide corporel.

2. Appareil selon la revendication 1, **caractérisé en ce que** les premiers moyens de pesage comprennent deux moyens de pesage indépendants (27, 28) pour peser respectivement le premier réservoir (10) et le second réservoir (17).

3. Appareil selon la revendication 1, **caractérisé en ce que** les premiers moyens de pesage comprennent des moyens de pesage (23) uniques pour peser ensemble le premier réservoir (10) et le second réservoir (17).

4. Appareil selon l'une des revendications 1 à 3, **caractérisé en ce que** les moyens d'extraction comprennent des quatrièmes moyens de pompage (26) pour faire circuler le sang dans le circuit pour circulation extracorporelle de sang (5, 7, 8), les troisièmes moyens de pompage (6) et les quatrièmes moyens de pompage (26) étant disposés respectivement en amont et en aval de l'échangeur (1).

5. Appareil selon l'une des revendications 1 à 3, **caractérisé en ce que** les moyens d'extraction comprennent des quatrièmes moyens de pompage (19) pour faire circuler le liquide usé.

6. Appareil selon l'une des revendications 1 à 5, **caractérisé en ce qu'**il comprend en outre des moyens de connexion (15, 16 ; 29) pour rendre le premier réservoir (10) sélectivement connectable à l'entrée du second compartiment (3) de l'échangeur (1) ou au circuit pour circulation extracorporelle de sang (5, 7, 8).

7. Appareil selon l'une des revendications 1 à 6, **caractérisé en ce qu'**il comprend des moyens de dosage (22, 25) pour ajuster à une concentration souhaitée $[A]_{DES}$ dans le sang du patient (9) la concentration d'une substance (A) présente dans le second liquide stérile, compte tenu du transfert diffusif et/ou convectif de la substance (A) au travers de la membrane (4) de l'échangeur (1).

8. Appareil selon la revendication 7, **caractérisé en ce que** les moyens de dosage comprennent des moyens (22, 25) pour régler le débit $(Q_A)$ du second liquide stérile en fonction du débit $(Q_{OUT})$ de liquide usé.

9. Appareil selon la revendications 8, **caractérisé en ce** le débit $(Q_A)$ du second liquide stérile et le débit $(Q_{OUT})$ du liquide usé sont liés par la formule :

$$Q_A = \frac{[A]_{DES}}{[A]_{SOL}} \times Q_{OUT}$$

où $[A]_{SOL}$ est la concentration de la substance (A) dans le premier liquide stérile.

10. Appareil selon la revendication 7, **caractérisé en ce que** les moyens de dosage comprennent des moyens (22, 25) pour régler le débit ($Q_A$) du second liquide stérile en fonction de la clairance Cl du rein artificiel pour la substance (A).

11. Appareil selon la revendications 10, **caractérisé en ce que** le débit ($Q_A$) du second liquide stérile et la clairance Cl du rein artificiel pour la substance (A) sont liés par la formule :

$$Q_A = \frac{[A]_{DES}}{[A]_{SOL}} \times Cl$$

où $[A]_{SOL}$ est la concentration de la substance (A) dans le premier liquide.

**Patentansprüche**

1. Multifunktionaler Apparat für die Zirkulation von körperlichen und medizinischen Flüssigkeiten in einem Austauscher (1), der zwei durch eine halbdurchlässige Membran (4) getrennte Kammern (2, 3) hat, wobei eine erste Kammer (2) mit einem an einen Patienten (9) anschließbaren Kreislauf (5, 7, 8) zur extrakorporalen Blutzirkulation verbunden ist, und eine zweite Kammer (3) einen Eingang und einen Ausgang hat, wobei dieser Apparat folgendes umfasst:

- erste Pumpvorrichtungen (12), um eine erste sterile Flüssigkeit zirkulieren zu lassen;
- zweite Pumpvorrichtungen (22), um eine zweite sterile Flüssigkeit zirkulieren zu lassen;
- dritte Pumpvorrichtungen (6, 26), um Blut im Kreislauf für extrakorporale Blutzirkulation (5, 7, 8) zirkulieren zu lassen;
- Extraktionsvorrichtungen (19, 26), um die Ultrafiltration einer körperlichen Flüssigkeit durch die Membran (4) des Austauschers (1) herbeizuführen;

wobei dieser Apparat **dadurch gekennzeichnet ist, dass** er Folgendes umfasst:

- erste Wiegevorrichtungen (23; 27, 28), um einen ersten Behälter (10) für die erste sterile Flüssigkeit und einen zweiten Behälter (17) für eine gebrauchte Flüssigkeit zu wiegen, wobei der erste Behälter (10) an den Eingang der zweiten Kammer (3) des Austauschers und der zweite Behälter (17) an den Ausgang der zweiten Kammer (3) des Austauschers (1) angeschlossen werden können;
- zweite Wiegevorrichtungen (24), um einen dritten Behälter (20) für die zweite sterile Flüssigkeit zu wiegen, wobei dieser Behälter an den extrakorporalen Blutkreislauf (5, 7, 8) angeschlossen werden kann; und
- Steuervorrichtungen (25) für den Empfang einer Gewichtsinformation von den ersten Wiegevorrichtungen (23; 27, 28) und einer Gewichtsinformation von den zweiten Wiegevorrichtungen (24), und um eine der Vorrichtungen unter den ersten, zweiten, dritten Pumpvorrichtungen (22, 12, 6, 26) und den Extraktionsvorrichtungen (19, 26) auf der Grundlage dieser Gewichtsinformationen und wenigstens einer Information bezüglich einer gewünschten Durchflussmenge wenigstens einer Flüssigkeit unter der ersten sterilen Flüssigkeit, der zweiten sterilen Flüssigkeit, dem Blut und der körperlichen Flüssigkeit zu steuern.

2. Apparat gemäß Anspruch 1, **dadurch gekennzeichnet, dass** die ersten Wiegevorrichtungen zwei unabhängige Wiegevorrichtungen (27, 28) umfassen, um jeweils den ersten Behälter (10) und den zweiten Behälter (17) zu wiegen.

3. Apparat gemäß Anspruch 1, **dadurch gekennzeichnet, dass** die ersten Wiegevorrichtungen eine einzige Wiegevorrichtung (23) umfassen, um den ersten Behälter (1) und den zweiten Behälter (17) zusammen zu wiegen.

4. Apparat gemäß einem der Ansprüche 1 bis 3, **dadurch gekennzeichnet, dass** die Extraktionsvorrichtungen vierte Pumpvorrichtungen (26) umfassen, um das Blut in dem Kreislauf für die extrakorporale Blutzirkulation (5, 7, 8) zirkulieren zu lassen, wobei die dritten Pumpvorrichtungen (6) und die vierten Pumpvorrichtungen (26) jeweils stromaufwärts und stromabwärts des Austauschers (1) angeordnet sind.

5. Apparat gemäß einem der Ansprüche 1 bis 3, **dadurch gekennzeichnet, dass** die Extraktionsvorrichtungen vierte Pumpvorrichtungen (19) umfassen, um die verbrauchte Flüssigkeit zirkulieren zu lassen.

6. Apparat gemäß einem der Ansprüche 1 bis 5, **dadurch gekennzeichnet, dass** er darüber hinaus Anschlussvorrichtungen (15, 16; 29) umfasst, um den ersten Behälter (10) selektiv an den Eingang der zweiten Kammer (3) des Austauschers (1) oder an den Kreislauf für die extrakorporale Blutzirkulation (5, 7, 8) anschließen zu können.

7. Apparat gemäß einem der Ansprüche 1 bis 6, **dadurch gekennzeichnet, dass** er Dosierungsvorrichtungen (22, 25) umfasst, um die Konzentration einer Substanz (A), die in der zweiten sterilen Flüssigkeit vorhanden ist, unter Berücksichtigung des diffusen und/oder konvektiven Ausschleusens der Substanz (A) durch die Membran (4) des Austauschers (1) an eine gewünschte Konzentration $[A]_{DES}$ im Blut des Patienten (9) anzupassen.

8. Apparat gemäß Anspruch 7, **dadurch gekennzeichnet, dass** die Dosierungsvorrichtungen Vorrichtungen (22, 25) umfassen, um die Durchflussmenge ($Q_A$) der zweiten sterilen Flüssigkeit in Abhängigkeit von der Durchflussmenge ($Q_{OUT}$) der verbrauchten Flüssigkeit zu regeln.

9. Apparat gemäß Anspruch 8, **dadurch gekennzeichnet, dass** die Durchflussmenge ($Q_A$) der zweiten sterilen Flüssigkeit und die Durchflussmenge ($Q_{OUT}$) der verbrauchten Flüssigkeit durch folgende Formel verbunden sind:

$$Q_A = \frac{[A]DES}{[A]SOL} \times Q_{OUT}$$

wobei $[A]_{SOL}$ die Konzentration der Substanz (A) in der ersten sterilen Flüssigkeit ist.

10. Apparat gemäß Anspruch 7, **dadurch gekennzeichnet, dass** die Dosierungsvorrichtungen Vorrichtungen (22, 25) umfassen, um die Durchflussmenge ($Q_A$) der zweiten sterilen Flüssigkeit in Abhängigkeit von der Clearance Cl der künstlichen Niere für die Substanz (A) zu regeln.

11. Apparat gemäß Anspruch 10, **dadurch gekennzeichnet, dass** die Durchflussmenge ($Q_A$) der zweiten sterilen Flüssigkeit und die Clearance Cl der künstlichen Niere für die Substanz (A) durch folgende Formel verbunden sind:

$$Q_A = \frac{[A]DES}{[A]SOL} \times Cl$$

wobei $[A]_{SOL}$ die Konzentration der Substanz (A) in der ersten Flüssigkeit ist.

**Claims**

1. A multifunction apparatus for circulating corporeal and medical liquids in an exchanger (1) having two compartments (2, 3), separated by a semipermeable membrane (4), a first compartment (2) being connected to an extracorporeal blood circuit (5, 7, 8) connectable to a patient (9), and a second compartment (3) having an inlet and an outlet, the apparatus comprising:

- first pumping means (12) for circulating a first sterile liquid;
- second pumping means (22) for circulating a second sterile liquid;
- third pumping means (6, 26) for circulating blood in the extracorporeal blood circuit (5, 7, 8);
- extraction means (10, 26) for causing ultrafiltration of a corporeal liquid through the membrane (4) of the exchanger (1);

the apparatus being **characterized in that** it comprises:

- first weighing means (23; 27, 28) for weighing a first reservoir (10) for the first sterile liquid, and a second reservoir (17) for a waste liquid, the first reservoir (10) being connectable to the inlet of the second compartment (3) of the exchanger, and the second reservoir (17) being connectable to the outlet of the second compartment (3) of the exchanger (1);
- second weighing means (24) for weighing a third reservoir (20) for the second sterile liquid, this reservoir being connectable to the extracorporeal blood circuit (5, 7, 8); and
- control means (25) for receiving a weight information from the first weighing means (23; 27, 28) and a weight information from the second weighing means (24) and for controlling one of the first, the second and the third pumping means (22, 12, 6, 26) and the extraction means (19, 26) from these weight information and from at least one information corresponding to a desired flow rate of at least one of the first sterile liquid, the second sterile liquid, the blood and the corporeal liquid.

2. An apparatus according to claim 1, **characterized in that** the first weighing means includes two independent weighing means (27, 28) for weighing the first reservoir (10) and the second reservoir (17), respectively.

3. An apparatus according to claim 1, **characterized in that** the first weighing means comprises a single weighing means (23) for weighing both the first reservoir (10) and the second reservoir (17).

**4.** An apparatus according to one of the claims 1 to 3, **characterized in that** the extraction means comprises fourth pumping means (26) for circulating blood in the extracorporeal blood circuit (5, 7, 8), the third pumping means (6) and the fourth pumping means (26) being disposed upstream and downstream of the exchanger (1) respectively.

**5.** An apparatus according to one of the claims 1 to 3, **characterized in that** the extraction means comprises fourth pumping means (19) for circulating the waste liquid.

**6.** An apparatus according to one of the claims 1 to 5, **characterized in that** it comprises connecting means (15, 16; 29) for permitting the first reservoir (10) to be selectively connectable to the inlet of the second compartment (3) of the exchanger (1) or to the extracorporeal blood circuit (5, 7, 8).

**7.** An apparatus according to one of the claims 1 to 6, **characterized in that** it comprises dosage means (22, 25) for adjusting the concentration of a substance (A), which is present in the second sterile liquid, to a desired concentration $[A]_{DES}$ in the blood of the patient (9), whereby the diffusive and/or convective transfer of the substance (A) through the membrane (4) of the exchanger (1) is taken into account.

**8.** An apparatus according to claim 7, **characterized in that** the dosage means comprises means (22, 25) for adjusting the flow rate ($Q_A$) of the second sterile liquid as a function of the flow rate ($Q_{OUT}$) of the waste liquid.

**9.** An apparatus according to claim 8, **characterized in that** the flow rate ($Q_A$) of the second sterile liquid and the flow rate ($Q_{OUT}$) of the waste liquid are related by the equation:

$$Q_A = \frac{[A]_{DES}}{[A]_{SOL}} \times Q_{OUT}$$

where $[A]_{SOL}$ is the concentration of the substance (A) in the first sterile liquid.

**10.** An apparatus according to claim 7, **characterized in that** the dosage means comprises means (22, 25) for adjusting the flow rate ($Q_A$) of the second sterile liquid as a function of the clearance Cl of the artificial kidney for the substance (A).

**11.** An apparatus according to claim 10, **characterized in that** the flow rate (QA) of the second sterile liquid and the clearance Cl of the artificial kidney for the substance (A) are related by the equation:

$$Q_A = \frac{[A]_{DES}}{[A]_{SOL}} \times Cl$$

where $[A]_{SOL}$ is the concentration of the substance (A) in the first liquid.

Fig. 1

Fig. 2